# EUROPEAN PATENT SPECIFICATION

(11) **EP 2 673 050 B1**
(45) Date of publication and mention of the grant of the patent: **30.03.2016**
(21) Application number: 11702480.2
(22) Date of filing: 10.02.2011
(51) Int. Cl.: A61P 37/00, C07K 14/415, A61K 38/17, A61K 38/16

(54) **PLANT HSP70 FOR USE IN THE TREATMENT OF FOOD ALLERGY**
HSP PFLANZLICHEN URSPRUNGS ZUR BEHANDLUNG VON NAHRUNGSMITTELALLERGIE
PLANTE HSP70 POUR UTILISATION DANS LE TRAITEMENT D'UNE ALLERGIE ALIMENTAIRE

(43) Date of publication of application: 18.12.2013
(73) Proprietor: Alfa Biogene International B.V., 48455 Bad Bentheim (DE)
(72) Inventor: LAX, Julia, 41379 Brüggen (DE); SEIFARTH, Federico G., 48455 Bad Bentheim (DE)
(74) Representative: V.O.
(86) International application number: PCT/EP2011/051989
(87) International publication number: WO 2012/107095

(56) References cited:
- WO-A1-2010/086418
- YAMASHITA YASUHIRO ET AL: "HSP70 inducers from Chinese herbs and their effect on melanin production", JOURNAL OF INVESTIGATIVE DERMATOLOGY, NATURE PUBLISHING GROUP, GB, vol. 130, no. Suppl.2, 1 September 2010 (2010-09-01), page S96, XP009148844, ISSN: 0022-202X
- SALUNGA THUCYDIDES L ET AL: "Identification of genes responsive to paeoniflorin, a heat shock protein-inducing compound, in human leukemia U937 cells", INTERNATIONAL JOURNAL OF HYPERTHERMIA, BASINGSTOKE, GB, vol. 23, no. 6, 1 September 2007 (2007-09-01), pages 529-537, XP009148848, ISSN: 0265-6736, DOI: 10.1080/02656730701639499

## Description

The invention is directed to plant Hsp70 or hydrolysate thereof for use in a treatment for food allergy and to a food product, inhaler or nasal spray comprising plant Hsp70 or a hydrolysate thereof.

Heat shock proteins (Hsp's) are a class of ubiquitous proteins synthesized in all life forms in response to stress. They are a natural product important for cell viability and cell repair.

An allergy is a hypersensitive disorder of the immune system. An allergy is one of four forms of hypersensitivity reactions and is called type I (or immediate) hypersensitivity. An allergy is caused by an adverse immune reaction to specific substances known as allergens.

Food allergy is an allergy caused by an allergic reaction to a substance ingested in food. In other words, a food allergy is an allergy wherein the allergen is a food component. In particular, a food protein, a food protein fragment or a food protein fraction may act as an allergen in food allergies. A food allergy should be distinguished from other adverse responses to food, such as food intolerance, pharmacological reactions and toxin-mediated reactions.

Allergens are identified by the organism and tagged with Immunoglobulin E (IgE). The immune system subsequently recognizes the tagged allergens as harmful and consequently triggers an allergic reaction. In an individual suffering from a food allergy, a generally harmless food protein is mistakenly identified by the body's immune system as harmful. The food protein thus acts as an allergen and may therefore also be referred to as an allergenic protein. Although strictly speaking, the allergy is caused by the allergenic protein, food allergies are generally named after the food which comprises the allergenic protein. For example, peanut allergy is an allergy caused by peanut protein.

Allergic responses caused by food allergy can range from mild responses, such as dermatitis, gastrointestinal and respiratory distress, to severe reactions, such as anaphylaxis, including biphasic anaphylaxis and vasodilation. Anaphylaxis is a life threatening systemic condition, causing a constriction of the trachea, preventing breathing, and anaphylactic shock.

Food allergies are a growing health concern with dramatic increase in the last two decades. More than 12 million US Americans suffer from food allergies. That's 4% of the population. The incidence of food allergy is highest in young and decreases with age.

Although there is a broad variety of known food allergens, over 90 % of adverse food reactions are caused by food components in the following foods: milk, eggs, peanuts, tree nuts, wheat, soy, fish, and shellfish. Other examples of food allergies are allergies caused by food components in legumes (soy, peas, beans), corn, maize, fruits, vegetables, spices, synthetic and natural colours, chicken and chemical additives.

One of the most common food allergies is peanut allergy. Peanuts belong to the family of legumes (*Fabaceae*). Peanut allergies may be extremely severe, but can sometimes be outgrown by children until school-age.

Proteins in tree nuts, including pecans, almonds, cashews, pistachios, pine nuts, and walnuts, are another widespread allergen. Subjects suffering from tree nut allergy may be sensitive to one, or many, tree nuts. Furthermore, seeds, including sesame seeds and poppy seeds, may contain oils comprising a protein that can act as an allergen.

Egg allergies affect about one in fifty children but are frequently outgrown by children when they reach the age of five. Typically, the sensitivity is to proteins in the egg white, rather than the yolk.

Allergy to milk from cows, goats or sheep seems to persist longer than other food allergies. Milk allergy is more common in children than in adults. It is estimated that 2 to 5 % of infants develop milk allergy. In 20% of affected children, the allergy persists until adulthood. Also, it is possible for adults to develop a milk allergy without having a history of allergies in their childhood. Many subjects suffering from milk allergy are also unable to tolerate dairy products such as cheese. Approximately 10% of children with a milk allergy may also develop an adverse reaction to beef as beef contains a small amount of protein present in cow's milk.

Lactose intolerance, a common reaction to milk, is not a form of allergy. Milk allergy is an adverse immune reaction to certain proteins in milk. Therefore, it may also be referred to as milk protein allergy. In contrast, lactose intolerance is a non-allergic food sensitivity caused by a lack of the enzyme lactase in the organism, such that the predominant sugar in milk cannot be sufficiently digested. Adverse effects of lactose intolerance generally occur after much higher levels of milk consumption compared to milk allergy, where even trace amounts of allergenic milk protein can cause a reaction.

Object of the invention is to provide a product for a treatment of a food allergy.

A further objective of the invention is to provide a product for treatment of specifically a peanut allergy or a milk allergy.

At least one of these objects is met by providing a plant Hsp70 or a hydrolysate thereof for use in a prophylactic and/or therapeutic treatment of a food allergy.

The subject to be treated may be an animal such as a mammal. Preferably, the subject to be treated is human. The subject may be an infant, a child, an adult or an elder.

The inventors found in animal experiments that the immune response to peanut extract in sensitized mice could be reduced after several days by peroral administration of isolated Hsp70 derived from alfalfa.

The inventors further contemplate that Hsp70 or hydrolysate thereof, in particular Hsp70 or hydrolysate thereof from alfalfa, is in particular suitable for treating cow's milk allergy.

An "allergen" as used herein refers to one molecule or a combination of molecules capable of provoking an immune response in a subject, in particular the production of an antibody against the molecule. In food allergy, the allergen is a food component, such as for example a food protein, a food protein fragment or a food protein fraction. An antibody typically produced is immunoglobulin E (IgE). An immune response where IgE is produced may be referred to as a type I hypersensitive response.

Heat shock proteins are formed by micro-organisms, plants and animals especially when, as a result of a change in the environment such as exposure to heat, radiation or chemicals, so-called stress-susceptible genes are expressed. According to current insights, such proteins can contribute to a protection against detrimental effects resulting from such environmental changes. The Hsp70s are an important part of the cell's machinery for protein folding and play a role in cell protection and repair of stress induced damage.

The food allergy may be selected from the group consisting of peanut allergy, milk allergy, nut allergy, corn allergy, fruit allergy, garlic allergy, oats allergy, shellfish allergy, soy allergy, wheat allergy (in particular gluten allergy), egg allergy, sesame allergy, olive oil allergy, cheese allergy, crustaceans allergy, fish allergy.

Plant Hsp70 was found to be particular effective for treating peanut allergy and milk allergy.

Milk allergy may be further distinguished by the animal from which the milk originates. Different milk allergies are for example cow's milk, goat's milk, sheep's milk, buffalo's milk, horse's milk and camel's milk allergy. In most case, a subject being allergic to one type of milk allergy will also be allergic to other types of milk allergy (although horse's milk allergy and camel's milk allergy are rare).

There are many potential protein allergens in milk that may cause allergic reactions. For example, cow's milk typically contains over 25 different proteins, which have the potential to elicit an allergic reaction. Casein and whey are the two main components. Casein typically accounts for about 80 percent of the protein in cow's milk and is the most important allergen found in many dairy products, for example milk and cheese. Whey typically accounts for about 20 percent of cow's milk. The two main allergenic proteins in whey are alpha-lactalbumin and beta-lactoglobulin. Consequently, the plant Hsp70 or hydrolysate thereof may be used more specifically for the treatment of casein protein allergy and/or whey protein allergy, such as alpha-lactalbumin allergy and beta-lactoglobulin allergy.

In a specific embodiment the plant Hsp70 or hydrolysate thereof is used for the prophylactic or therapeutic treatment of a child that has a milk allergy. Up to 60% of infants allergic to cow's milk will outgrow the allergy by the age of 4 and 80% by the age of 6. However, a significant percentage of subjects suffering from milk allergy remains into adulthood. Also, it is possible for adults to develop a milk allergy without having a history of allergies in their childhood. Therefore, the plant Hsp70 or hydrolysate thereof may also be used for the treatment of an adult.

The allergic reaction may comprise many symptoms. Possible symptoms of food allergy include skin reactions (such as inflammation of the skin, anaphylaxis, hives, eczema, swelling of lips, mouth tongue, face, throat and/or skin), stomach and intestinal reactions (such as abdominal pain and bloating, diarrhoea, vomiting, gas, cramps), cardiovascular reactions (vasodilation, blood pressure drop, microvascular leakage), and reactions of the airways, in particular of the nose, threat and/or lungs, (such as sneezing, watery and/or itchy eyes, coughing, broncho-constriction, wheezing, shortness of breath, asthma). The invention provides a plant Hsp70 or hydrolysate thereof for use in a treatment comprising the treatment of one or more of these allergic reactions. In particular, Hsp70 or a hydrolysate thereof is suitable for use in a treatment comprising the treatment of anaphylaxis or dermatitis.

The plant Hsp70 or hydrolysate thereof may be administered by a method selected from the group consisting of enteral administration, inhalation administration, parenteral administration, mucosal administration, rectal administration (administration through the rectum) and topical administration (for example application to the skin).

Preferably, the plant Hsp70 or hydrolysate thereof is administered by mucosal administration. Mucosal administration is the administration through mucosal surfaces, such as through the mucosa of the nose (intranasal administration) or through the mucosa of the mouth (oral administration). Examples of such oral administration are sublingual administration (under the tongue) and buccal administration (in the cheek). In particular, intranasal administration is preferred. Mucosal administration has the advantage that the Hsp70 or the hydrolysate thereof is directly absorbed into the bloodstream. This is a quick and efficient way of administration. Furthermore, this has the advantage that Hsp70 or the hydrolysate thereof does not pass the gastrointestinal tract and/or the liver circulation, thus lowering the chance of Hsp70 or the hydrolysate thereof being damaged or degraded before being effective.

The Hsp70 or a hydrolysate thereof according to the invention may be administered by enteral administration. Enteral administration as used herein refers to the introduction of a product into the stomach or intestines, such as by tube feeding or by peroral administration (such as swallowing). Surprisingly, enterally administered plant Hsp70 or hydrolysate thereof introduced into the stomach via a tube, has been found to be effective in the treatment of a disease that is not a disease of the gastro-intestinal tract, *viz.* a food allergy. Short-term access is usually achieved using nasogastric (NG) or nasojejunal (NJ) tubes at an initial continuous feeding rate of 20-40, such as about 30 mls per hour. Percutaneous endoscopic gastrotomy (PEG) or jejunostomy placement should be considered if feeding is planned for longer than one month.

In particular, enteral administration may refer to the introduction of a product into the stomach or intestines via a tube, for example a transnasal, transoral or precutaneous tube. Preferably, enteral administration is done by intragastric gavage, which is the introduction of a product directly into the stomach via a tube. Examples of intragastric gavage are percutaneous gavage, wherein the product is administered into the stomach through a tube which has surgically been placed through the skin into the stomach, nasogastric gavage, wherein a product is administered via a tube that is inserted through the nose, past the throat, and down into the stomach or orogastric gavage, wherein a tube is passed through the mouth past the esophagus into the stomach. Examples of suitable tubes, also called feeding tubes, are percutaneous gastric feeding tubes, nasogastric feeding tubes, orogastric feeding tubes, duodenal feeding tubes and jejunal feeding tubes.

The plant Hsp70 or hydrolysate thereof according the invention may also be administered via parenteral administration. Parenteral administration as used herein refers to the administration of plant Hsp70 or hydrolysate thereof by means of injection, such as injection into a vein (intravenous administration), into a muscle (intramuscular administration) or under the skin (subcutaneous administration).

It is contemplated that enteral feeding may offer advantages over parenteral feeding, including lower cost, greater convenience, decreased infectious complications, and enhanced host immune function. Another beneficial effect includes improved maintenance of gastro-intestinal mucosal structure and function, which could possibly prevent gut atrophy and bacterial translocation. On the other hand, in some embodiments, such as the treatment of certain medical conditions, parenteral feeding may be the first choice of administration. For example, in case of decreased function of the gastro-intestinal tract or presence of medical reasons to bypass it, Hsp70 or a hydrolysate thereof can be administered parenterally. Another advantage of parenteral administration lies in its quick distribution in the body over the blood stream.

The Hsp70 or a hydrolysate thereof according to the invention may further be administered by application to the skin, for example in a solution or emulsion comprising plant Hsp70 or hydrolysate thereof. The solution or emulsion may be aqueous, oil-based or a combination thereof. When applied to the skin, the Hsp70 or a hydrolysate thereof is thought to be absorbed through and into the skin where it displays its therapeutic or prophylactic action. The Hsp70 may for example be applied via a patch.

In case the plant Hsp70 or hydrolysate thereof is to be administered by mucosal administration, the plant Hsp70 or hydrolysate is preferably administered via inhalation. In this administration method, the Hsp70 or hydrolysate thereof are applied as part of a suspension into the airway. The suspension may be a suspension of solid, liquid or combined solid-liquid particles comprising Hsp70, which particles are suspended in a fluid. For example, the suspension may be a suspension of solid Hsp70, Hsp70 present in an oil phase or Hsp70 dispersed in water or a combination thereof. The fluid may be any pharmaceutically acceptable fluid.

The Hsp70 or hydrolysate thereof, dissolved or suspended in a liquid phase, may be provided in a container further comprising a propellant, in particular a propellant gas under pressure, which can be released as a fine spray. Preferably, the suspension is an aerosol, in particular a suspension of fine solid particles or liquid droplets in a gas. In case of an aerosol, the fluid of the suspension is a gas. The gas may be any pharmaceutically acceptable gas, for example air. Preferably, the plant Hsp70 or hydrolysate thereof is present in the aerosol as a dry freezed powder. The aerosol is preferably prepared by suspending dry freezed Hsp powder in a gas under pressure. Accordingly, the invention also relates to an aerosol comprising plant Hsp or hydrolysate thereof and a spray, in particular an aerosol spray, comprising a container holding plant Hsp70 or hydrolysate thereof.

An aerosol can be inhaled directly into the lungs via a mouthpiece or face mask. The respiratory effort transports the suspension via the airways to the lung tissue where it is absorbed into the capillary bed. Advantage of this route of administration is the above mentioned avoidance of the gastrointestinal tract and/or the liver circulation (known as the first pass effect) and direct administration into the bloodstream and/or target organ (e.g. airways and lungs). After gastrointestinal absorption, the majority of the blood (including absorbed nutrients and medications) passes the liver where pharmaceutical agents can be metabolized and rendered inactive. By inhaling or absorption over nasal mucosa this first pass effect can be avoided, such that lower dosages reach the same bioavailability.

As described above, the invention is also directed to a spray, in particular an aerosol spray, comprising a container holding plant Hsp70 or hydrolysate thereof. The aerosol spray functions by releasing the plant Hsp70 or hydrolysate as a fine spray, in particular as an aerosol. The aerosol spray may further comprise a propellant, in particular a propellant gas under pressure, which can be released as a fine spray.

The spray may be a nasal spray. A nasal spray provides a suitable instrument to administer the Hsp70 or hydrolysate thereof intranasally. The nasal spray may further comprise a nozzle for spraying the Hsp70 or hydrolysate thereof into a nostril. The nasal spray may operate by releasing the plant Hsp70 or hydrolysate thereof into a nostril, in particular as part of an aerosol. In an embodiment, the nasal spray comprises an isotonic saline solution comprising Hsp70 or hydrolysate thereof. Such a solution may be prepared by dissolving plant Hsp70 or hydrolysate thereof in a saline solution, subsequently optionally adjusting the solution to a physiologic pH using a pharmaceutical grade buffer and subsequently optionally diluting the solution to an isotonic solution.

The aerosol spray may be an inhaler. An inhaler provides a suitable instrument to administer the Hsp70 or hydrolysate thereof through the mucosa of the mouth (orally) and/or the lungs. The inhaler may further comprise a mouthpiece for inhaling the aerosol. The inhaler may operate by releasing the plant Hsp70 or hydrolysate thereof into the mouth, in particular as an aerosol.

The suspension comprising plant Hsp70 or hydrolysate thereof, as defined above, may be administered using the spray of the invention.

The application of an aerosol according to the invention may comprise pumping pre-mixed fluid comprising Hsp70 from a holding tank to a spray head, and then mixing the fluid with a stream of rapidly moving air or gas. The air stream may divide the Hsp70 containing fluid into small droplets and delivers it to the site of action, i.e. the nasal mucosa. The properties of the fluid are preferably such that the fluid can get into the nostrils without condensing ("fogging out") and/or evaporate easily.

The plant Hsp70 or hydrolysate thereof of the invention may also be administered as a food product and may for example be selected from the group consisting of dairy products, dairy-substitute products, fruit products, soft-drinks, confectionary (e.g. chocolate), cereal products (e.g. pasta, grenola) and infant formulas. In particular, the plant Hsp70 or hydrolysate thereof may be administered as a medical nutrition. The term medical nutrition as used herein, is used for a food product that is intended to be administered other than by normal oral ingestion (peroral administration), such as in particular by parenteral administration or via enteral feeding (e.g. tube feeding). Generally, medical nutrition is liquid, e.g. the medical nutrition may be an emulsion, a suspension or a syrup. An example of a medical nutrition is an enteral tube feeding. Preferably, no chewing is required to suitably consume the medical nutrition. Furthermore, the medical nutrition may be a dry powder which can be used as a nutritional supplement. Such a powder may for example be dissolved or suspended in water directly before consuming.

The plant Hsp70 or hydrolysate thereof of the invention may in particular be administered as part of a food product comprising an allergen capable of causing a food allergy. Consequently, the invention is also directed to a food product comprising plant Hsp70 or hydrolysate thereof and an allergen capable of causing a food allergy. Such a food product is desirable, because subjects suffering from food allergy may not always be able to digest a sufficient amount of the nutritional compounds that are typically present in the food they are allergic to. By providing a food product with both the allergen and Hsp70, a subject suffering from food allergy will be able to eat food to which he is allergic and does not have to change its diet because of the food allergy. Thus, the invention contributes to a more varied and healthy diet.

For example, the intake of dairy products in young children is generally considered to be healthy. Children with milk allergy may not always be able to digest a sufficient amount of the nutritional compounds present in dairy products. However, a dairy product comprising plant Hsp70 or hydrolysate thereof will not cause the allergic reaction and can thus be consumed by children having milk allergy. Such a dairy product may for example be selected from the group of dairy products, such as yoghurt, cheese, milk, yoghurt-drinks, ice-cream other milk-based desserts and butter.

The plant Hsp70 or hydrolysate thereof may be administered in a dosage of at least 1 µm, for example at least 10 µg, at least 50 µg or at least 100 µg per day. Furthermore, the plant Hsp70 or hydrolysate thereof may be administered in a daily dosage of 10000 µm or less, for example 1000 µg or less, 500 µg or less or 250 µg or less.

These dosages are suitable for a subject to be treated in general. However, the specific dosages depend both on the subject (weight, age) and on the administration method.

For example, a dosage for an adult will typically be lower than a dosage for an infant or a child.

Furthermore, a preferred dosage may depend on the administration method used. For example, a dosage of the Hsp70 or hydrolysate thereof in mucosal and rectal administration will generally be lower for achieving a similar effect compared to enteral administration, but will generally be higher compared to parenteral administration. Consequently, a preferred daily dosage will generally be lower when using mucosal (such as intranasal) administration than when using enteral administration, but higher than when when using parenteral administration.

The dosage of the Hsp70 or hydrolysate thereof may further depend on the body weight of the subject. The plant Hsp70 or hydrolysate thereof may be administered in a dosage of at least 0.5 µg per kg body weight per day (µg/kg/day), for example at least 1, at least 5, at least 10 or at least 20 µg/kg/day. The plant Hsp70 or hydrolysate thereof may be administered in a dosage of 1000 µg /kg/day or less, for example 500 µg/kg/day or less, 250 µg/kg/day or less or 100 µg/kg/day or less.

The total concentration of the Hsp 70 plus hydrolysate thereof in a product of the invention (*i.e.* spray, aerosol or food product, such as medical nutrition, may be chosen within wide limits. Generally, said total concentration is up to 0.1 wt.% based on the total weight of the product, usually in the range of 1 to 1 000 µg per 100 g product.

The total concentration of the Hsp 70 plus hydrolysate thereof per 100 g product may in particular be 10 µg or more, more in particular 25 µg or more, or 50 µg or more. The total concentration of the Hsp 70 plus hydrolysate thereof per 100 g product may be 1 000 µg or less, in particular 500 µg or less, more in particular 250 µg or less.

For a medical nutrition the total concentration of the Hsp 70 plus hydrolysate thereof may be in the range of 1-1000 µg/100 ml, preferably 25-250 µg/100 ml.

In a specific embodiment the food product is a medical nutrition (suitable or intended for administration to an infant), wherein the total concentration of the Hsp 70 plus hydrolysate thereof may in particular be chosen in the range of 1-1000 µg/100 ml, preferably 50-100 µg per 100 ml.

In particular in a food product for oral consumption, such as a dairy product, a dairy substitute product, a fruit product or a soft drink, the concentration may be in the range of 1-1000 µg/100 ml, preferably 50-250 µg/100 g product.

In terms of the dosage of plant Hsp70 or hydrolysate thereof per administration (for bolus administration), this (bolus) dosage may be 1-1000 µg. Preferably it is in the range of 1-250 µg, in particular 10-150 µg, more in particular 20-80 µg.

The Hsp70 may be used in the prophylactic treatment of food allergy. To provide a subject suffering from food allergy with sufficient resistance to a food allergy each day, the subject may be administered the above-mentioned dosage at least once a week, more preferably at least three times a week or once a day. The dosage may also be used prior to or during consuming the food to which the subject is allergic to avoid an allergic response.

The Hsp70 may further be used in the therapeutic treatment of food allergy. In this case, the Hsp70 is to be administered after the allergic reaction takes place, in particular in a dosage as described above each day, until the allergic reaction has stopped. The invention is further directed to a food product comprising plant Hsp70 or hydrolysate thereof, in particular alfalfa Hsp, or a hydrolysate thereof. Such a food product may be packaged in a unit dosage packaging and comprises a total dosage of Hsp70 of 1-250 µg, in particular 10-150 µg, more in particular 20-80 µg.

A food product of the invention may comprise a carbohydrate fraction, a protein fraction, a lipid fraction and optionally additives. The carbohydrate fraction may provide 30-60 %, preferably 40-50 %, of the total energetic value of the food product (en%). The protein fraction may provide 5-25, preferably 10-20 en%. The lipid fraction may provide 25-55 en%, preferably 35-45 en%. Proteins are composed of amino acids. A total of 20 proteinogenic amino acids have been identified, of which nine are essential in infants. Those are preferably contained in food products of the invention, in particular in enteral or parenteral food products, such as feeding solutions.

The greatest part of the human body's caloric needs is generally supplied by carbohydrates. Sugars are common components of enteral feeding solutions, such as glucose or dextrose, which may be used in standard parenteral solutions. The carbohydrate fraction of the food product of the invention may for example comprise a sugar selected from the group consisting of glucose and dextrose.

Lipids are another major source of calories. The lipid fraction of the food product may for example comprise straight-chained fatty acids, saturated and non-saturated, varying in length from 4 to 24 carbon atoms. Since humans do not synthesize linoleic and alpha-linolenic acid, these can be present in the lipid fraction as well. The lipid fraction of the food product may further comprise soy oil, egg components and/or glycerin, in particular in parenteral lipid solutions. If the food product is an enteral feeding solution, the lipid fraction may comprise vegetable oils which provide a variety of fatty acids.

The additive may be selected from the group consisting of electrolytes, trace elements and minerals. Minerals, vitamins and trace elements may for example be present in enteral and parenteral feeding solutions. Their amount can be modified in order to satisfy special needs or replace ongoing losses.

The Hsp70 in the invention may be provided in the form of a microparticle. Consequently, the invention is also directed to a microparticle comprising plant Hsp70.

As used herein, microparticles include micro- or nanoscale particles. Usually, the outer surface of the particles is composed of solid or semi-solid material. Typically, the average diameter of the microparticles given by the Fraunhofer theory in volume percent ranges from 10 nm to 1000 µm. The preferred average diameter depends on the intended use. For instance, in particular in case the microparticles are intended for parenteral administration, an average diameter of up to 10 µm may be desired.

It is envisaged that microparticles with an average diameter of less than 800 nm, in particular of 500 nm or less, are useful for intracellular purposes. For such purposes, the average diameter preferably is at least 20 nm or at least 30 nm.

In another embodiment, larger dimensions may be desirable, for instance a diameter in the range of 1-100 µm or 10-100 µm. It is envisaged that in particular for enteral feed tubing such a relatively large diameter may be advantageous: in such embodiment factors such as unpleasant mouth feel due to a large particle size do not play a role, and a relatively large size may help to allow at least a substantial part of the Hsp70 or hydrolysate thereof to pass the stomach without being (further) degraded by the gastric fluid, which may be undesired in at least some embodiments.

In particular, the particle diameter as used herein is the diameter as determined by a LST 230 Series Laser Diffraction Particle size analyzer (Beckman Coulter), making use of a UHMW-PE (0.02 - 0.04 µm) as a standard. Particle-size distributions are estimated from Fraunhofer diffraction data and given in volume (%).

If the particles are too small or non analyzable by light scattering because of their optical properties then scanning electron microscopy (SEM) or transmission electron microscopy (TEM) can be used.

The microparticle structure may be a substantially homogenous structure, including nano- and microspheres and the like. In an embodiment, the microparticles essentially consist of Hsp70 or hydrolysate thereof. The microparticle may for example comprise at least 95 wt.% or at least 99 wt.% plant Hsp70 or hydrolysate thereof.

The microparticles may comprise an encapsulating material.

The encapsulating material may function as a carrier material. In this case, microparticles are prepared by mixing the Hsp70 or hydrolysate thereof with the encapsulating material. The encapsulating material may in this case also be referred to as carrier material. A microparticle comprising a carrier material and Hsp70 or hydrolysate thereof may for instance be desirable when a specific release profile of Hsp70 or hydrolysate thereof into the intestines or into the blood is needed.

The encapsulating material may also function as a shell material. In this case, the microparticle comprises an inner nucleus comprising the Hsp or hydrolysate thereof and an outer shell comprising the carrier or encapsulating material.. The content of the Hsp70 or hydrolysate thereof is preferably higher in the nucleus than in the shell. The content of the encapsulating material is preferably higher in the shell than in the nucleus. The provision of such a shell around a nucleus comprising the Hsp70 or hydrolysate thereof may be advantageous in that it may protect the Hsp70 or hydrolysate against damage and/or degradation during storage and/or after administration. Damage during storage may for example be caused by interaction with the surroundings during storage, such as with other food ingredients, with water in the food product and/or with oxygen in the food product. Damage after administration may for example occur when the Hsp70 is administrated through the gastrointestinal tract, which environment may damage and/or degrade the Hsp70. The shell may in particular protect the Hsp70 or hydrolysate thereof against gastric juice (in particular in case of an enteric shell or coating).

Suitable encapsulating materials are known in the art and may for instance be selected from (medical) food grade biomolecules such as monosaccharides, disaccharides, oligosaccharides, digestible polysaccharides, non-digestible polysaccharides, proteins, fats and other lipids or (medical) food grade synthetic molecules. In particular, the encapsulating material may be a molecule that can safely degrade or dissolve in the gastro-intestinal tract. The encapsulating material may further improve the release of the Hsp or hydrolysate thereof into the blood or into the target tissue (if any), which is particular desirable for food products, in particular parenteral food products.

The encapsulating material may more specifically be selected from the group consisting of gum acacia, maltodextrins, hydrophobically modified starch, alginate, carrageenan, pectin, guar gum, gum acacia, locust bean gum, gellan gum, agar, dextran, glucose, cyclodextrins, cellulose derivatives (such as carboxymethyl cellulose, methyl cellulose, ethyl cellulose), wax, paraffin, beeswax, diacyl glycerols, vegetable or animal oils/fats, whey proteins, soy proteins, sodium caseinate, gelatin, gluten, albumin,

For example, Hsp or hydrolysate thereof may be encapsulated in a liposome, e.g. in a phospolipid liposome. It is contemplated that this may in particular be interesting, in case encapsulation is desired for a parenteral nutritional product.

In a specific embodiment, the encapsulating material may be an enteric coating, which is at least substantially resistant to the gastric juice, but which physically or chemically degrades in the intestines, to release the Hsp or hydrolysate thereof. Enteric coatings are known in the art, *e.g.* an enteric coating may comprise at least one component selected from the group consisting of cellulose acetate phthalate, cellulose acetate butylate phthalate, methyl acrylate-methacrylic acid copolymers, cellulose acetate succinate, hydroxy propyl methyl cellulose phthalate, hydroxy propyl methyl cellulose acetate succinate (hypromellose acetate succinate), polyvinyl acetate phthalate and methyl methacrylate-methacrylic acid copolymers. Eudragit® is an example of a commercially available enteric encapsulating material.

In a specific embodiment, the Hsp or hydrolysate thereof is encapsulated in food-grade material, that remains at least substantially undissolved and undegraded under gastric conditions, but that is dissolved or degraded in the intestine or affected in an other manner that causes the particles to disintregrate and release the Hsp or hydrolysate thereof.

Suitable methods of preparing microparticles are known in the art. The many available technologies for microencapsulation can roughly be divided into two categories, one which uses a liquid as a suspending medium (complex coacervation, interfacial and in situ polymerization or solvent evaporation from emulsions) and one which uses a gas as a suspending medium into which a liquid phase is sprayed (spray-drying, spray-cooling or spray-chilling, fluidized-bed coating or coextrusion). Both methods are suitable to prepare the microparticles of the invention. The skilled person will know how to prepare microparticles according to these methods.

The microparticles may for example be prepared by spray-cooling and spray-chilling. These techniques involve dispersing the water-soluble ingredient in a molten encapsulating material, such as fat or wax, and spraying this dispersion through heated nozzles into a chamber at ambient temperature (spray-cooling) or at refrigeration temperatures (spray-chilling). If the chamber is at room temperature, the encapsulation material usually has a melting point between 45 and 122°C. If the chamber is cooled, materials melting at 32-42°C can be used.

The microparticles may also be prepared by spray drying, which is a microencapsulation technique widely used in the food industry. It is also applicable to encapsulating material that have no melting point or that have a melting point that is above the maximum temperature to which the HSP or hydrolysate should be exposed. The process is economical; flexible, in that it offers substantial variation in microencapsulation matrix; adaptable to commonly used processing equipment; and produces particles of good quality. Usually the Hsp or Hsp-hydrolysate is mixed with the encapsulating material in water or another suitable carrying liquid that can be removed from the (precursors of) the microparticles by drying. Examples of encapsulating materials that have been used in spray dry process (not necessarily with Hsp or Hsp-hydrolysate) include gum acacia, maltodextrins, hydrophobically modified starch, alginate, carboxymethylcellulose, guar gum, glucose (corn syrup), dextran, pectin, gellan gum, agar and proteins (whey proteins, soy proteins, sodium caseinate).

Furthermore, microparticles may be prepared by coacervation, which technique is known in the prior art, also in relation to food technology, as for example described in WO 2007/026307 (using starch/starch hydrolysate).

Further, encapsulating methods that may be employed to provide microparticles for a food product according to the invention are, e.g., based on WO 2001/000233 (using alginate as encapsulating material), U.S. Patent No. 4,232,047 (using e.g starch, protein (gelatin), flour, modified starch, gum, or mixtures thereof) or U.S. Patent No. 4,230,687 (a controlled-release product prepared by dispersing an active agent in an encapsulant, such as modified starch, a gum, or a protein material such as gelatine or casein).

Plant Hsp70 or hydrolysate thereof refers to Hsp70 obtained from a plant or a hydrolysate thereof. The plant Hsp70 or hydrolysate thereof of the invention may be a Hsp70 or hydrolysate thereof obtained from a plant selected from the group of cereals (for instance barley), soy, grasses (for instance oat), beet, potato, clover and water plants (for instance an alga). Preferably, the plant is alfalfa. Preferably the source is a harvested plant material. In particular, roots, flowers, stems, leaves, more in particular the leaves of the plant may be used as source for Hsp70. Particularly suitable are beet tops, alfalfa leaves, barley leaves, oat leaves, and potato tops.

Preferably, the plant Hsp70 is a natural, *i.e.* non-recombinant, Hsp70; the Hsp70-hydrolysate preferably is a hydrolysate from natural (non-recombinant) Hsp70. It is contemplated that a natural Hsp70 or hydrolysate of natural Hsp may be tolerated better by a subject treated with the plant Hsp70, in particular that the risk of allergenic reactions may be less.

A product according to the invention may comprise native plant Hsp70 or denatured plant Hsp70.

The plant Hsp70 or hydrolysate thereof used in the invention may be full length plant Hsp70. Full length plant Hsp70 as defined herein is in particular a plant Hsp70 that has at least 90%, more in particular 95%, even more in particular 99% of the peptide length of the same type of the Hsp70 found in the plant from which the Hsp70 originates. Full length plant Hsp70 may still have lipid groups and/or sugar groups attached to the protein, which may affect the efficiency of plant Hsp70 in the treatment of food allergy.

Plant Hsp70 or hydrolysate thereof from alfalfa is particularly preferred. Hsp70 or hydrolysate thereof from alfalfa has a high similarity with Hsp70 from humans, making it particularly suitable for use in the treatment of humans. For example, the peptide segments relevant for immune response in Hsp70 from alfalfa shows, in contrast with Hsp70 from other organisms, no differences in amino acid sequence between human HSP70, as can be seen in Table 1 and Table 2.

Non-recombinant Hsp70 from plant may comprise full posttranslational modifications (e.g. acetylation, hydroxylation, glycosylation, amino acid addition, alkylation) capable to increase efficacy in the treatment of food allergy.

The product according to the invention may be hydrolysed Hsp70. The degree of hydrolysis may be chosen within wide limits. At least 10 wt. %, at least 25 wt. %, at least 50 wt. %, at least 80 wt. % or at least 90 wt. % (based on the sum of HSP-fragment and unhydrolysed HSP) of the HSP-hydrolysate may be formed by HSP fragments. Of the HSP-hydrolate 100 wt. % or less, 95 wt. % or less, at least 75 wt. % or less, 50 wt. % or less or 25 wt. % or less (based on the sum of HSP-fragments and unhydrolysed HSP) may be formed by HSP fragments.

The size of the fragments may be chosen within wide limits. Usually, in case a hydrolysate is present, at least 50 wt. %, in particular at least 75 wt. %, more in particular at least 90 wt. % (based on the sum of HSP-fragments and unhydrolysed HSP) of the hydrolysate is formed by peptides (including unhydrolysed HSP) having at least five amino acid residues. In a specific embodiment, at least 25 wt. %, in particular at least 50 wt. %, more in particular at least 75 wt. % (based on the sum of HSP-fragments and unhydrolysed HSP) of the hydrolysate is formed by peptides (including unhydrolysed HSP) having at least ten amino acid residues.

Usually, in case a hydrolysate is present, at least 10 wt. %, in particular at least 25 wt. %, more in particular at least 50 wt. % (based on the sum of HSP-fragments and unhydrolysed HSP) of the hydrolysate is formed by HSP fragments.

Plant Hsp70 or hydrolysate thereof from alfalfa is particularly preferred. Hsp70 from alfalfa has a high similarity with Hsp70 from humans, making it particularly suitable for use in the treatment of humans. For example, the peptide segments relevant for immune response in Hsp70 from alfalfa shows, in contrast with Hsp70 from other organisms, no differences in amino acid sequence between human HSP70, as can be seen in Table 1 and Table 2.

**Table 1: first segment:**

| | | |
|---|---|---|
| Human¹ | LNVLRIINEPTAAAIAYGLD | #differences human SEQ |
| Alfalfa² | LNVLRIINEPTAAAIAYGLD | 0 |
| Mycobacterium | LNVLRIVNEPTAAALAYGLD | 2 |
| Maize | LNVMRIINEPTAAAIAYGLD | 1 |
| Tobacco | LNVMRIINEPTAAAIAYGLD | 1 |
| Tomato | LDVLRIINEPTAASLAYGFE | 5 |
| Wheat | LRVLRIINEPTAAAIAYGLD | 1 |

| | | |
|---|---|---|
| ¹ amino acid positions 167-186 ² amino acid positions 171-190 | | |

**Table 2: second segment**

| | | |
|---|---|---|
| Human³ | NPDEAVAYGAAVQAAIL | #differences human SEQ |
| Alfalfa⁴ | NPDEAVAYGAAVQAAIL | 0 |
| Mycobacterium | NPDEVVAVGAALQAGVL | 5 |
| Maize | NPDEAVAYGAAVQAAIL | 0 |
| Tobacco | NPDEAVAYGAAVQAAIL | 0 |
| Tomato | NPDEVVALGASVQAGIL | 4 |
| Wheat | NPDEAVAYGASVQAAIL | 1 |

| | | |
|---|---|---|
| ³ amino acid positions 364-380 ⁴ amino acid positions 370-386 | | |

The invention will further be illustrated by the following experimental examples.

### Example 1: Effect of Hsp70 on Peanut Allergy

Experiments were done using three groups (A-C), each group comprising eight mice. Each group first received a pretreatment on days -10, - 7, -5 and -3. Subsequently, each group received a sensitization treatment on days 0, 1, 2, 7, 14, 21 and 28. On day 35, each group were administered peanut extract (PE) intragastrically via a tube into the stomach.

Group A received phosphate buffered saline (PBS) via intragastric gavage as the pretreatment and cholera toxin (CT) in PBS as the sensitization treatment. Group A was also referred to as the negative control group.

Group B received PBS via intragastric gavage as the pretreatment and peanut extract and CT in PBS as the sensitization treatment. Group B was also referred to as the peanut allergic group.

Group C received alfalfa Hsp70 in PBS via intragastric gavage as the pretreatment and peanut extract and CT in PBS as the sensitization treatment. Group B was also referred to as the Hsp70 treated group.

Furthermore, at day 23, blood was collected from the cheek of the mice in each group.

Furthermore, at day 36 section was conducted on each mouse.

The immune response of the mice to the peanut extract was measured by measuring the concentration of Immunoglobulin G1 (IgG1), Immunoglobulin G2a (IgG2a) and Immunoglobulin E (IgE) antibodies in the blood obtained from the mice at day 23. Furthermore, the mouse mast cell protease-1 (mMCP-1) antibody was measured. Mast cells are primary mediators of allergic inflammation. Antigen-mediated crosslinking of IgE receptors on mast cell surfaces results in degranulation and the release of pro-inflammatory mediators such as histamine, tumor necrosis factor-a, and/or leukotrienes. Thus, mMCP-1 is a parameter for mast cell degranulation and, consequently, allergic inflammation.

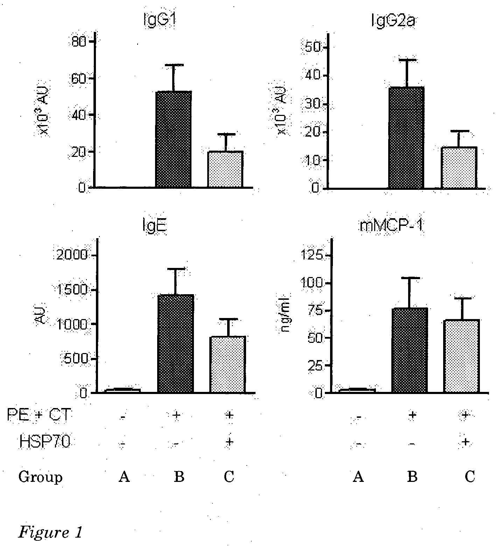

The results of the measurements are depicted in Figure 1. The left bar in each graph represents the negative control group (group A), the middle bar the peanut allergic group (group B) and the right bar the Hsp70 treated group (group C).

Figure 1 shows that all four antibody concentrations measured showed a lower concentration for group C compared to group B. From this it can be concluded that the immune response to peanut extract was much lower in mice treated with alfalfa Hsp70 (group C) compared to untreated mice of group B.

### Example 2: Food Product

In a hospital setting, parenteral or enteral nutrition can be composed and adapted to the needs of every patient. Standard solutions cover the average daily requirements of fluids and nutrients.

In healthy toddlers, the following dosages may serve as guideline to compose a parenteral or enteral formula to meet an energy requirement of 80 to105 kcal/kg/day. Energy and fluid requirements change over time. Hence, the quantities need to be adjusted in older and younger children, as in sick patients with special nutritional needs.
Fluid requirement: 100ml/day
Carbohydrates: 12mg/kg/min: 45% of calories
Protein: 2.5-3g/kg/day: 15% of calories
Lipids: 3g/kg/day: 40% of calories
Additives: Electrolytes, trace elements, minerals
Kilocalorie requirements: 80-105 ckal/kg/day

## Claims

1. Plant Hsp70 or a hydrolysate thereof for use in a prophylactic or therapeutic treatment of a food allergy, wherein the food allergy is a legume allergy.

2. Hsp70 or a hydrolysate thereof for use according to claim 1, wherein the plant is alfalfa.

3. Hsp70 or a hydrolysate thereof for use according to claim 1 or 2, wherein the legume allergy is peanut allergy.

4. Hsp70 or a hydrolysate thereof for use according to any of the previous claims, wherein the treatment of the food allergy comprises the treatment of an allergic reaction selected from the group consisting of anaphylaxis and dermatitis.

5. Hsp70 or a hydrolysate thereof for use according to any of the previous claims, wherein the Hsp70 or hydrolysate thereof is to be administered mucosally, preferably intranasally.

6. Hsp70 or a hydrolysate thereof for use according to any of the previous claims, wherein the Hsp70 or hydrolysate thereof is to be administered by inhalation.

7. Hsp70 or a hydrolysate thereof for use according to any of claims 1-4, wherein the Hsp70 or hydrolysate thereof is to be administered enterally, e.g. perorally, or parenterally.

8. Hsp70 or a hydrolysate thereof for use according to any of claims 1-4 or 7, wherein the Hsp70 is to be administered as a food product, preferably a food product selected from the group consisting of dairy products, dairy-substitute products, fruit products, soft-drinks, confectionary (*e.g.* candy, chocolate), cereal products (*e.g.* baked products, pasta) and infant formulas.

9. Hsp 70 or a hydrolysate thereof for use according claim 8, wherein the food product comprises an allergen capable of causing the food allergy to be treated in a subject suffering said allergy.

10. Hsp70 or a hydrolysate thereof for use according to claim 8 or 9, wherein the Hsp70 is to be administered as a medical nutrition.

11. Hsp70 or a hydrolysate thereof for use according to any of the previous claims, wherein the Hsp70 is to be administered in a dosage of 1-250 µg, in particular 10-150 µg, more in particular 20-80 µg.

12. Hsp70 or a hydrolysate thereof for use according to any of the previous claims, wherein the Hsp70 or hydrolysate thereof is to be administered as part of a microparticle.

13. Food product comprising plant Hsp70 or hydrolysate thereof, which food product further comprises a carbohydrate fraction, a protein fraction and a lipid fraction, wherein
the carbohydrate fraction provides 30-60 en%, preferably 40-50 en%,
the protein fraction provides 5-25 en%, preferably 10-20 en%, and
the lipid fraction provides 25-55 en%, preferably 35-45 en%, and wherein the food product comprises an allergen capable of causing a food allergy, wherein the food allergy is a legume allergy.

14. Food product according to claim 13, wherein the food product is packaged in a unit dosage packaging and comprises a total dosage of Hsp70 of 1-250 µg, in particular 10-150 µg, more in particular 20-80 µg.

15. Aerosol suitable for oral or intranasal administration, comprising a plant Hsp70 or a hydrolysate thereof.

16. Nasal spray comprising a container holding plant Hsp70 or hydrolysate thereof and a nozzle for spraying the suspension.

17. Inhaler comprising a container holding a plant Hsp70 or hydrolysate thereof and a mouthpiece for inhaling.

## Patentansprüche

1. Hsp70 pflanzlichen Ursprungs oder ein Hydrolysat davon zur Verwendung bei einer prophylaktischen oder therapeutischen Behandlung einer Nahrungsmittelallergie, wobei die Nahrungsmittelallergie eine Hülsenfruchtallergie ist.

2. Hsp70 oder ein Hydrolysat davon zur Verwendung nach Anspruch 1, wobei die Pflanze Luzerne ist.

3. Hsp70 oder ein Hydrolysat davon zur Verwendung nach Anspruch 1 oder 2, wobei die Hülsenfruchtallergie eine Erdnussallergie ist.

4. Hsp70 oder ein Hydrolysat davon zur Verwendung nach einem der vorhergehenden Ansprüche, wobei die Behandlung der Nahrungsmittelallergie die Behandlung einer allergischen Reaktion umfasst, ausgewählt aus der Gruppe bestehend aus Anaphylaxie und Dermatitis.

5. Hsp70 oder ein Hydrolysat davon zur Verwendung nach einem der vorhergehenden Ansprüche, wobei das Hsp70 oder Hydrolysat davon mukosal, bevorzugt intranasal, verabreicht werden soll.

6. Hsp70 oder ein Hydrolysat davon zur Verwendung nach einem der vorhergehenden Ansprüche, wobei das Hsp70 oder Hydrolysat davon durch Inhalation verabreicht werden soll.

7. Hsp70 oder ein Hydrolysat davon zur Verwendung nach einem der Ansprüche 1-4, wobei das Hsp70 oder Hydrolysat davon enteral, *z*. *B.* peroral, oder parenteral, verabreicht werden soll.

8. Hsp70 oder ein Hydrolysat davon nach einem der Ansprüche 1-4 oder 7, wobei das Hsp70 als Nahrungsmittelprodukt verabreicht werden soll, bevorzugt ein Nahrungsmittelprodukt, ausgewählt aus der Gruppe bestehend aus Milchprodukten, Milchersatzprodukten, Obstprodukten, Erfrischungsgetränken, Süßwaren (*z. B.* Bonbons, Schokolade), Getreideprodukten (*z. B.* Backwaren, Pasta) und Säuglingsnahrung.

9. Hsp70 oder ein Hydrolysat davon zur Verwendung nach Anspruch 8, wobei das Nahrungsmittelprodukt ein Allergen umfasst, das in der Lage ist, die Nahrungsmittelallergie, die behandelt werden soll, bei einem Patienten, der an dieser Allergie leidet, herbeizuführen.

10. Hsp70 oder ein Hydrolysat davon zur Verwendung nach Anspruch 8 oder 9, wobei das Hsp70 als eine medizinische Ernährung verabreicht werden soll.

11. Hsp70 oder ein Hydrolysat davon zur Verwendung nach einem der vorhergehenden Ansprüche, wobei das Hsp70 in einer Dosierung von 1-250 µg, bevorzugt 10-150 µg, bevorzugter 20-80 µg verabreicht werden soll.

12. Hsp70 oder ein Hydrolysat davon zur Verwendung nach einem der vorhergehenden Ansprüche, wobei das Hsp70 oder Hydrolysat davon als Teil eines Mikropartikels verabreicht werden soll.

13. Nahrungsmittelprodukt, umfassend Hsp70 pflanzlichen Ursprungs oder ein Hydrolysat davon, wobei das Nahrungsmittelprodukt ferner einen Kohlehydratanteil, einen Proteinanteil und einen Lipidanteil umfasst, wobei
der Kohlehydratanteil 30-60 Energie%, bevorzugt 40-50 Energie% liefert,
der Proteinanteil 5-25 Energie%, bevorzugt 10-20 Energie% liefert, und der Lipidanteil 25-55 Energie%, bevorzugt 35-45 Energie% liefert, und wobei das Nahrungsmittelprodukt ein Allergen umfasst, das in der Lage ist, eine Nahrungsmittelallergie herbeizuführen, wobei die Nahrungsmittelallergie eine Hülsenfruchtallergie ist.

14. Nahrungsmittelprodukt nach Anspruch 13, wobei das Nahrungsmittelprodukt in einer Einheitsdosisverpackung verpackt ist und eine Gesamtdosis Hsp70 von 1-250 µg, bevorzugt 10-150 µg, bevorzugter 20-80 µg, umfasst.

15. Aerosol, geeignet zur oralen oder intranasalen Verabreichung, umfassend ein Hsp70 pflanzlichen Ursprungs oder ein Hydrolysat davon.

16. Nasenspray, umfassend einen Behälter mit Hsp70 pflanzlichen Ursprungs oder ein Hydrolysat davon und eine Düse zum Versprühen der Suspension.

17. Inhalator, umfassend einen Behälter mit einem Hsp70 pflanzlichen Ursprungs oder ein Hydrolysat davon und ein Mundstück zum Inhalieren.

## Revendications

1. Hsp70 d'origine végétale ou hydrolysat de celle-ci destiné(e) à être utilisé(e) dans un traitement prophylactique ou thérapeutique d'une allergie alimentaire, dans laquelle l'allergie alimentaire est une allergie aux légumineuses.

2. Hsp70 ou hydrolysat de celle-ci pour son utilisation selon la revendication 1, dans laquelle la plante est la luzerne.

3. Hsp70 ou hydrolysat de celle-ci pour son utilisation selon la revendication 1 ou 2, dans laquelle l'allergie aux légumineuses est une allergie aux arachides.

4. Hsp70 ou hydrolysat de celle-ci pour son utilisation selon l'une quelconque des revendications précédentes, dans laquelle le traitement de l'allergie alimentaire comprend le traitement d'une réaction allergique choisie dans le groupe constitué par l'anaphylaxie et la dermatite.

5. Hsp70 ou hydrolysat de celle-ci pour son utilisation selon l'une quelconque des revendications précédentes, dans laquelle l'Hsp70 ou son hydrolysat est destiné(e) à être administré(e) par voie muqueuse, de préférence intranasale.

6. Hsp70 ou hydrolysat de celle-ci pour son utilisation selon l'une quelconque des revendications précédentes, dans laquelle l'Hsp70 ou son hydrolysat est destiné(e) à être administré(e) par inhalation.

7. Hsp70 ou hydrolysat de celle-ci pour son utilisation selon l'une quelconque des revendications 1 à 4, dans laquelle l'Hsp70 ou son hydrolysat est destiné(e) à être administré(e) par voie entérale, p. *ex.* par voie perorale, ou parentérale.

8. Hsp70 ou hydrolysat de celle-ci pour son utilisation selon l'une quelconque des revendications 1 à 4 ou 7, dans laquelle l'Hsp70 est destinée à être administrée sous la forme d'un produit alimentaire, de préférence un produit alimentaire choisi dans le groupe constitué par les produits laitiers, les produits de substitution aux produits laitiers, les produits à base de fruits, les boissons non alcoolisées, les confiseries (*p. ex.* bonbons, chocolat), les produits à base de céréales (*p. ex.* produits cuits, pâtes) et les formules pour nouveau-nés.

9. Hsp70 ou hydrolysat de celle-ci pour son utilisation selon la revendication 8, dans laquelle le produit alimentaire comprend un allergène capable de provoquer l'allergie alimentaire qui doit être traitée chez un sujet souffrant de ladite allergie.

10. Hsp70 ou hydrolysat de celle-ci pour son utilisation selon la revendication 8 ou 9, dans laquelle l'Hsp70 est destinée à être administrée sous forme d'une nutrition médicale.

11. Hsp70 ou hydrolysat de celle-ci pour son utilisation selon l'une quelconque des revendications précédentes, dans laquelle l'Hsp70 est destinée à être administrée à une dose de 1 à 250 µg, en particulier de 10 à 150 µg, et mieux encore de 20 à 80 µg.

12. Hsp70 ou hydrolysat de celle-ci pour son utilisation selon l'une quelconque des revendications précédentes, dans laquelle l'Hsp70 ou son hydrolysat est destiné(e) à être administré(e) sous forme de partie de microparticule.

13. Produit alimentaire comprenant l'Hsp70 d'origine végétale ou un hydrolysat de celle-ci, ledit produit alimentaire comprenant en outre une fraction glucidique, une fraction protéique et une fraction lipidique, dans lequel
la fraction glucidique fournit 30 à 60 % de la valeur énergétique, de préférence 40 à 50 %,
la fraction protéique fournit 5 à 25 % de la valeur énergétique, de préférence 10 à 20 %, et
la fraction lipidique fournit 25 à 55 % de la valeur énergétique, de préférence 35 à 45 %, et
dans lequel le produit alimentaire comprend un allergène capable de provoquer une allergie alimentaire, ladite allergie alimentaire étant une allergie aux légumineuses.

14. Produit alimentaire selon la revendication 13, dans lequel le produit alimentaire est conditionné dans un emballage unitaire et comprend une dose totale d'Hsp70 de 1 à 250 µg, en particulier de 10 à 150 µg, et mieux encore de 20 à 80 µg.

15. Aérosol se prêtant à une administration par voie orale ou intranasale, comprenant une Hsp70 d'origine végétale ou un hydrolysat de celle-ci.

16. Pulvérisateur à usage nasal comprenant un récipient renfermant l'Hsp70 d'origine végétale ou son hydrolysat et une buse pour pulvériser la suspension.

17. Inhalateur comprenant un récipient renfermant une Hsp70 d'origine végétale ou son hydrolysat et un embout buccal pour l'inhalation.
